# EUROPEAN PATENT SPECIFICATION

(11) **EP 3 363 784 B1**
(45) Date of publication and mention of the grant of the patent: **28.10.2020**
(21) Application number: 17156549.2
(22) Date of filing: 16.02.2017
(51) Int. Cl.: C07C 291/04, C11D 1/75

(54) **DIMETHYL FARNESYL AMINE OXIDE AND ITS USE AS SURFACTANT OR WETTING AGENT**
DIMETHYLFARNESYLAMINOXID UND DESSEN VERWENDUNG ALS TENSID ODER BENETZUNGSMITTEL
N-OXYDE DE DIMÉTHYLFARNESYLAMINE ET SON UTILISATION COMME SURFACTANT OU PRODUIT MOUILLANT

(43) Date of publication of application: 22.08.2018
(73) Proprietor: Clariant International Ltd, 4132 Muttenz (CH)
(72) Inventor: Leinweber, Dirk, 65779 Kelkheim (DE); Romanski, Steffen, 46483 Wesel (DE); Guo, Xiaoqiang, 65929 Frankfurt am Main (DE); Vorholt, Andreas, 44137 Dortmund (DE); Behr, Arno, 44137 Dortmund (DE); Faßbach, Thiemo Alexander, 45276 Essen (DE)
(74) Representative: Clariant Produkte (Deutschland) GmbH

(56) References cited:
- FR-A1- 2 320 280
- GB-A- 2 032 422
- GB-A- 2 297 973
- US-A- 5 679 633
- VICTORIA G SÃ NCHEZ ET AL: "Synthesis, Surface-Active Properties, and Anthelmintic Activities of New Cationic Gemini Surfactants Against the Gastrointestinal Nematode,, In Vitro", JOURNAL OF SURFACTANTS AND DETERGENTS, SPRINGER/OACS, USA, vol. 15, no. 4, 8 March 2012 (2012-03-08), pages 463-470, XP035068162, ISSN: 1558-9293, DOI: 10.1007/S11743-012-1337-0
- Kunihiko Takabe ET AL: "A SIMPLE CONVERSION OF N , N -DIMETHYL-2-ALKENYLAMINE TO 2-ALKENAL", Chemistry Letters, vol. 11, no. 12, 5 December 1982 (1982-12-05), pages 1987-1988, XP055569618, JAPAN ISSN: 0366-7022, DOI: 10.1246/cl.1982.1987

## Description

The present invention relates to amine oxides comprising farnesyl residues (dimethyl farnesyl amine oxides) and compounds obtainable by reacting a dimethyl farnesyl amine with hydrogen peroxide in the presence of carbon dioxide, to a process for the preparation of these amine oxides or compounds, to their uses as surfactants or wetting agents, and to crop protection compositions, hard surface cleaning compositions, laundry detergent compositions, and automatic dishwashing compositions comprising one or more of these amine oxides or compounds.

Trialkyl amine oxides are well-known as surfactants and foaming agents that are used in a variety of applications, including sanitizers, cleaners, emulsifiers, fabric softeners, oil drilling lubricants, and the like. The particular application for which a given amine oxide will be preferred depends upon its functional characteristics, which in turn depend upon the nature of the alkyl substituents. The functional properties include surface tension reduction, wetting ability, and the amount and quality of the foam produced. Structural parameters include the number of long chain alkyl groups, their length and their degree of branching.

US 5,679,633 discloses low foaming branched alkyl dimethyl amine oxides synthesized from branched fatty alcohol based on oil refinery products.

US 2011/034363 describes highly branched trialkyl amine oxides which are synthesized from polyunsaturated and polybranched hydrocarbons such as farnesene by a multi-step process. The preparation of these surfactants e.g. starts with a hydroformylation of the polyunsaturated and polybranched hydrocarbons to the corresponding aldehydes. These aldehydes are subsequently hydrogenated to the fully saturated alcohols, followed by halogenation, amination and oxidation. The resulting amine oxides are said to have an enhanced foaming ability.

GB 2 297 973 discloses processes for synthesizing mono-long chain amine oxide surfactants.

Victoria G SANCHEZ ET AL: "Synthesis, Surface-Active Properties and Anthelmintic Activities of New Cationic Gemini Surfactants Against the Gastrointestinal Nematode, In Vitro", JOURNAL OF SURFACTANTS AND DETERGENTS; SPRINGER/OACS, USA, vol. 15, no. 4, 8 March 2012, pages 463-470 discloses N,N-dimethylfarnesylamine and its conversion to cationic dimeric surfactants.

FR 2 320 280 discloses N,N-dimethylnerylamine and its N-oxide and N,N diethylnerylamine and its N-oxide and processes for the synthesis thereof.

GB 2 032 422 discloses N,N-diethylerylamine and its N-oxide and process for synthesis thereof.

Kunihiko Takabe ET AL: "A SIMPLE CONVERSION OF N,N-DIMETHYL-2-ALKENYLAMINE TO 2-ALKENAL", Chemistry Letters, vol. 11, no. 12, 5 December 1982, pages 1987-1988 discloses the synthesis of 2-alkenal from N,N-dimethyl-2-alkenylamine.

However, there is still a big need for low-foaming or non-foaming renewable substances that can be prepared in an atom economic way, exhibit superior surface tension reduction and wetting properties, and may be used in various applications, e.g. as crop protection adjuvant, dishwasher rinse aid, hard surface cleaning detergent or laundry detergent additive.

Therefore, it was the object of the present invention to provide low-foaming or non-foaming renewable substances that can be prepared in an atom economic way, and exhibit superior surface tension reduction and wetting properties.

Surprisingly, it was found that this object is solved by the amine oxides of the formula (I) 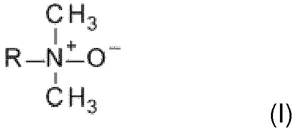 wherein
R is a radical of the formula 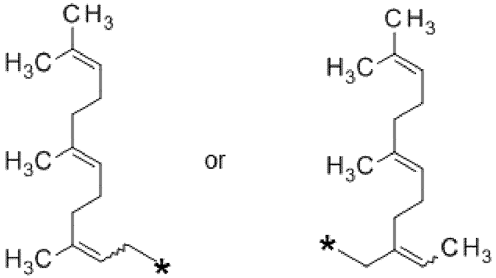
* indicates the binding site and
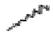 is a bond and indicates that the double bond connected thereto can be either cis or trans in configuration.

Therefore, one subject matter of the present invention is amine oxides of the formula (I) 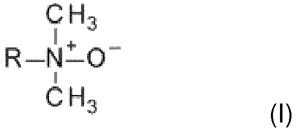 wherein
R is a radical of the formula 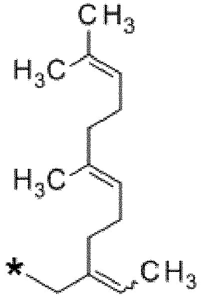 wherein
   * indicates the binding site and
   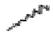 is a bond and indicates that the double bond connected thereto can be either cis or trans in configuration
   or
R is a radical of the formula 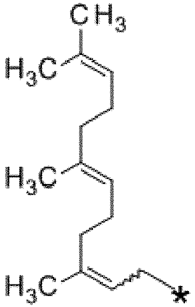 wherein
   * indicates the binding site and
   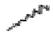 is a bond and indicates that the double bond connected thereto is cis in configuration.

The inventive amine oxides of the formula (I) are dimethyl farnesyl amine oxides. The inventive dimethyl farnesyl amine oxides (as well as the inventive compounds described further below) provide advantageous surface tension reduction and wetting characteristics while producing no foam or no stable foam.

The inventive dimethyl farnesyl amine oxides of the formula (I) may be synthesized from dimethyl farnesyl amine by oxidation with hydrogen peroxide in the presence of carbon dioxide (oxidation reaction). The dimethyl farnesyl amine may be obtained by reacting farnesene with dimethyl amine or with a dimethyl amine source (hydroamination reaction). The starting farnesene is 100% renewable. Even starting from farnesene, the process enables to produce the inventive amine oxides (as well as the inventive compounds described further below) in only two steps, and is highly atom economic. The product is highly renewable.

The inventive amine oxides (as well as the inventive compounds described further below) have an advantageous water-solubility.

As already stated above, the inventive dimethyl farnesyl amine oxides of the formula (I) may be synthesized from dimethyl farnesyl amine by oxidation with hydrogen peroxide in the presence of carbon dioxide.

A further subject matter of the present invention therefore is a process for the preparation of amine oxides described above, comprising reacting a dimethyl farnesyl amine with hydrogen peroxide in the presence of carbon dioxide.

The oxidation of alkyl amines to alkyl amine oxides, and in particular using hydrogen peroxide in the presence of carbon dioxide (which may be used as such or which may be generated from a carbon dioxide source such as sodium bicarbonate), is a reaction well known to the person skilled in the art (see e.g. US 5,866,718).

Preferably, the oxidation reaction to prepare the inventive amine oxides or inventive compounds is performed in a solvent, more preferably in a solvent comprising water and one or more further solvents different from water, and even more preferably in water as the solvent.

Preferred further solvents different from water for the oxidation reaction to prepare the inventive amine oxides or inventive compounds are selected from the group consisting of organic liquids in which the tertiary amine and tertiary amine oxide are soluble at the reaction temperature and which are capable of forming an azeotrope with water. However, to avoid the danger of explosion, these solvents should be substantially inert.

Particularly preferred further solvents different from water for the oxidation reaction to prepare the inventive amine oxides or inventive compounds are the lower alkyl alcohols, such as the C₁₋₈ alcohols, and especially the C₁₋₄ alcohols, containing one or more hydroxyl groups. Exemplary alcohols include methanol, ethanol, 1-propanol, 2-propanol, 1-butanol, 2-butanol, 1-pentanol, 2-pentanol, 3-pentanol, tert-butyl alcohol, 1-hexanol, 2-hexanol, 3-hexanol, 2-methyl-1-propanol, 2-methyl-2-propanol, tert-amyl alcohol, 2-methyl-1-butanol, 3-methyl-1-butanol, 3-methyl-2-butanol, neopentyl alcohol, 2,3-dimethyl-2-butanol, 3,3-dimethyl-2-butanol, 1-hexyl alcohol, 2-hexanol, 3-hexanol, and the like as well as various mixtures thereof. Especially preferred solvents include 1-propyl alcohol, 2-propyl alcohol, 1-butanol, and 2-butanol.

Preferably, the oxidation reaction to prepare the inventive amine oxides or inventive compounds is performed at a temperature of from 20 to 100 °C and more preferably at a temperature of from 30 to 70 °C.

Preferably, the oxidation reaction to prepare the inventive amine oxides or inventive compounds is performed at atmospheric pressure.

Preferably, in the oxidation reaction to prepare the inventive amine oxides or inventive compounds, the molar ratio dimethyl farnesyl amine : hydrogen peroxide is of from 1 : 1 to 1 : 50, preferably of from 1 : 1 to 1 : 10 and even more preferably of from 1 : 1 to 1 : 3.

Preferably, the oxidation reaction to prepare the inventive amine oxides or inventive compounds may be performed in the presence of chelating agents, e.g. diethylenetriamine-pentaacetic acid or salts thereof, such as the pentasodium salt, ethylenediaminetetraacetic acid (EDTA) or salts thereof, in order to protect the hydrogen peroxide from decomposition caused by metal ions.

In a preferred embodiment of the invention the dimethyl farnesyl amines are those wherein the double bond located closest to the dimethyl amine functional group is trans in configuration.

In another preferred embodiment of the invention the dimethyl farnesyl amines are mixtures of head and tail products without any selectivity on the trans or cis configuration of the double bond located closest to the dimethyl amine functional group.

As already stated above, the dimethyl farnesyl amine used in the oxidation reaction to prepare the inventive amine oxides or inventive compounds may be obtained by reacting farnesene with dimethyl amine or a dimethyl amine source in a hydroamination reaction.

The hydroamination of unsaturated hydrocarbons to respective amines, and in particular using dimethyl amine or a dimethyl amine source, is a reaction well known to the person skilled in the art. (see e.g. J. Pawlas; Y. Nakao.; M. Kawatsura; J. F. Hartwig. A general nickel-catalyzed hydroamination of 1,3-dienes by alkylamines: Catalyst selection, scope, and mechanism. J. Am. Chem. Soc. 2002, 124 (14), 3669-3679.).

Preferably, the dimethyl farnesyl amine used in the oxidation reaction to prepare the inventive amine oxides or the inventive compounds is obtained by reacting farnesene with dimethyl amine or a dimethyl amine source in the presence of a transition metal catalyst.

Preferably, the dimethylamine source used in the hydroamination reaction to prepare dimethyl farnesyl amine is selected from the group consisting of dimethyl amine hydrochloride, preferably to be used together with a base, and dimethyl ammonium dimethyl carbamate.

The hydroamination reaction to prepare dimethyl farnesyl amine may be performed in diverse solvents, such as in organic aliphatic or aromatic solvents, preferably in polar or polar aprotic hydrocarbons or ionic liquids. Particularly preferred are e.g. the following solvents:
- Aliphatic hydrocarbons, such as n-pentane, n-hexane, n-octane, iso-octane;
- Alcohols, such as methanol, ethanol, iso-propanol, tert-butanol;
- Aromatic hydrocarbons, such as toluene, xylene, mesitylene;
- Nitriles, such as acetonitrile, 3-methoxy propionitrile;
- Amides, such as dimethyl formamide, dimethylacetamide;
- Ethers, such as diethyl ether, methyl-tert-butyl ether, anisole;
- Carbonates, such as ethylene-, propylene- or butylene carbonate;
- Ionic liquids, such as dimethyl ammonium dimethyl carbamate (DimCarb);
- Highly polar solvents, such as dimethyl sulfoxide, N-methyl pyrrolidone.

Even more preferred solvents for the hydroamination reaction to prepare dimethyl farnesyl amine are anisole, dimethyl formamide, dimethylacetamide, methanol, iso-propanol, dioxane, dimethyl sulfoxide (DMSO), dimethyl ammonium dimethyl carbamate (DimCarb) and acetonitrile.

Depending on the dimethyl amine source used, the hydroamination reaction to prepare dimethyl farnesyl amine may also be performed without additional solvent. This is e.g. the case when DimCarb is used, because in this case the DimCarb is used as solvent and also as a reactant.

In a preferred embodiment of the present invention, the weight ratio solvent: reactants in the hydroamination reaction to prepare dimethyl farnesyl amine is 1 : 1 to 15 : 1, preferably 2 : 1 to 10 : 1 and more preferably 3 : 1 to 5 : 1.

As catalyst in the hydroamination reaction to prepare dimethyl farnesyl amine, transition metals selected from group 10 of the periodic table and preferably selected from the group consisting of nickel-, palladium- and platinum-precursors, are used.

Therefore, the transition metal catalyst for the hydroamination reaction to prepare dimethyl farnesyl amine preferably comprises a metal selected from group 10 of the periodic table and more preferably comprises a metal selected from the group consisting of nickel, palladium and platinum.

In the hydroamination reaction to prepare dimethyl farnesyl amine the transition metal catalyst is completely dissolved in the reaction mixture and is modified by an organic ligand.

The catalyst and the farnesene are used in the hydroamination reaction to prepare dimethyl farnesyl amine preferably in a molar ratio catalyst: farnesene of from 1:10 to 1:1000, more preferably of from 1:10 to 1:200, and even more preferably of from 1:10 to 1:125.

Preferred precursors are selected from the following group:
- Nickel precursors, such as Ni⁰(cod)₂, Ni^{II}(acac)₂, Ni^{II}(hfacac)₂, Ni^{II}Cl₂;
- Palladium precursors, such as Pd⁰₂dba₃, Pd^{II}(acac)₂, Pd^{II}(hfacac)₂, Pd^{II}(tfa)₂, Pd^{II}Cl₂;
- Platinum precursors, such as Pt^{II}Cl₂, Pt^{II}(codCl₂, Pt^{II}(acac)₂, K₂Pt^{II}Cl₄.

Particularly preferred are palladium precursors with fluorinated leaving groups, such as Pd^{II}(tfa)₂ or Pd^{II}(hfacac)₂.

In a preferred embodiment of the invention the catalyst used in the hydroamination reaction to prepare dimethyl farnesyl amine may be recycled after the reaction.

One possibility for the separation of the catalyst is the use of a polar phase and a catalyst with polar ligands for the preparation of nonpolar products. The nonpolar products may then be extracted with nonpolar hydrocarbons such as n-decane or n-dodecane. The catalyst remains in the polar phase and may be used for further reactions.

In the case of using ammonium carbamates for the hydroamination reaction to prepare dimethyl farnesyl amine, such as dimethylammonium dimethylcarbamate (DimCarb), and suitably sulfonated ligands for the catalyst, such as TPPMS, TPPTS or DPPBTS, the catalyst complex may be immobilized in the polar phase and may be separated from the product that forms a distinct phase that may optionally be extracted with additional DimCarb. The catalyst may be used for further reactions, e.g. by adding fresh farnesene to the polar phase and starting a new hydroamination reaction to prepare dimethyl farnesyl amine.

The transition metal catalyzed hydroamination reaction to prepare dimethyl farnesyl amine may be performed either with or without ligands. Preferably, phosphor ligands are used. The following list comprises some selected examples for ligands:
Triphenylphosphane (PPh₃);
sodium 3-(diphenylphosphanyl)benzenesulfonate (TPPMS);
sodium 3,3',3"-phosphanetriyltribenzenesulfonate (TPPTS);
Triphenyl phosphite (P(OPh)₃);
tris(2-methoxyphenyl)phosphane (TOMPP);
Tricyclohexylphosphane (PCy₃);
Triethylphosphane (PEt₃);
Tri-*tert*-butylphosphane (P*t*Bu₃);
1,2-bis(diphenylphosphanyl)ethane (DPPE);
1,3-bis(diphenylphosphanyl)propane (DPPP);
1,4-bis(diphenylphosphanyl)butane (DPPB);
sodium 3,3',3",3'''-(butane-1,4-diylbis(phosphanetriyl))tetrabenzenesulfonate (DPPBTS);
1,7-bis(diphenylphosphanyl)heptane (DPPH);
1,1'-bis(diphenylphosphino)-ferrocene (DPPF);
(9,9-dimethyl-9H-xanthene-4,5-diyl)bis(diphenylphosphane) (XantPhos);
sodium 4,5-bis(diphenylphosphanyl)-9,9-dimethyl-9H-xanthene-2,7-disulfonate (Sulfo-XantPhos);
4,6-bis(diphenylphosphanyl)-10H-phenoxazin (NiXantPhos);
(oxybis(2,1-phenyl))bis(diphenylphosphane) (DPEPhos).

Preferably, the molar ratio metal: ligand (e.g. palladium : ligand) is of from 1:1 to 1:50, more preferably of from 1:2 to 1:30, and even more preferably of from 1:2 to 1:16.

Preferably, the molar ratio farnesene : (dimethylamine or dimethyl amine source) in the hydroamination reaction to prepare dimethyl farnesyl amine is of from 1:1 to 1:10 and more preferably of from 1:2 to 1:5.

Preferably, the hydroamination reaction to prepare dimethyl farnesyl amine is performed in the presence of an inert gas, more preferably in the presence of argon or nitrogen.

Preferably, the hydroamination reaction to prepare dimethyl farnesyl amine is performed at a pressure of from 1 to 10 bar, more preferably at a pressure of from 2 to 8 bar, and even more preferably at a pressure of from 3 to 6 bar.

Preferably, the hydroamination reaction to prepare dimethyl farnesyl amine is performed at a temperature of from 50 to 150 °C, more preferably at a temperature of from 60 to 120 °C, and even more preferably at a temperature of from 70 to 100 °C;

In the context of the present invention "farnesene" is understood to encompass alpha-farnesene and beta-farnesene. Among those beta-farnesene is preferred. Farnesene is commercially available.

The inventive amine oxides and the inventive compounds are advantageously suited as surfactants and preferably as low-foaming surfactants, furthermore preferably in crop protection applications, hard surface applications, laundry applications or automatic dishwashing.

Therefore, a further subject matter of the present invention is the use of amine oxides of formula (I) 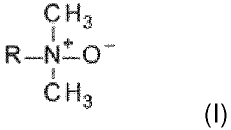 wherein
R is a radical of the formula 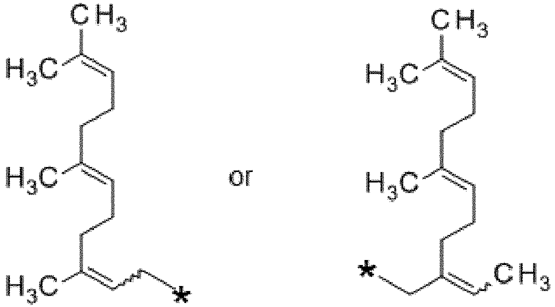
* indicates the binding site and
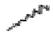 is a bond and indicates that the double bond connected thereto can be either cis or trans in configuration,
preferably an amine oxide of formula (Ia) 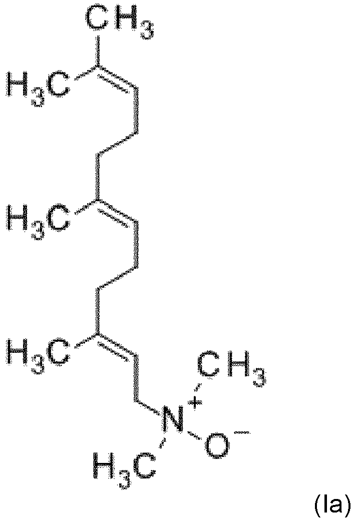 as surfactants and preferably as low-foaming surfactants, furthermore preferably in crop protection applications, hard surface applications, laundry applications or automatic dishwashing.

The inventive amine oxides and the inventive compounds are furthermore advantageously suited as wetting agents, preferably in crop protection applications, hard surface applications or laundry applications.

Therefore, a further subject matter of the present invention is the use of amine oxides of formula (I) 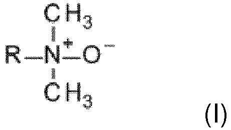 wherein
R is a radical of the formula 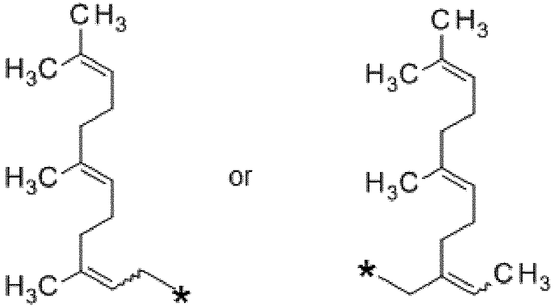
* indicates the binding site and
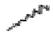 is a bond and indicates that the double bond connected thereto can be either cis or trans in configuration,
preferably an amine oxide of formula (Ia) 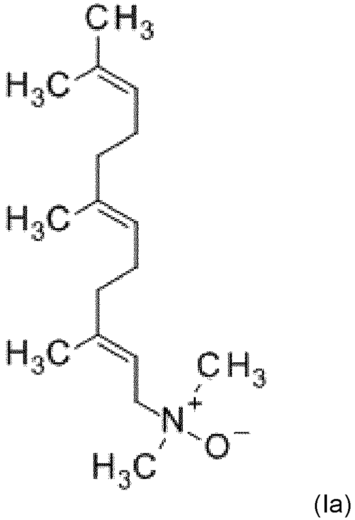 as wetting agents, preferably in crop protection applications, hard surface applications or laundry applications.

The inventive amine oxides or inventive compounds can be blended with other substances to provide formulations useful in a variety of industrial and other applications in which low foam, high surface tension reduction, and fast wetting times are desired. Non-limiting examples of such substances include alkalizing agents, amphoteric surfactants, quaternary ammonium compounds, sequestering agents, dyes and fragrances. In general, these agents should not contribute significant amounts of foam to the formulation. In the case of (inventive amine oxide or inventive compound)/amphoteric surfactant blends, the respective weight ratio inventive amine oxide : amphoteric surfactant in the blend is generally between 5:1 and about 1:5.

Examples of alkalizing agents that can be used with the inventive amine oxides or inventive compounds include sodium hydroxide, sodium carbonate and sodium metasilicate.

A further subject matter of the present invention are crop protection compositions, hard surface cleaning compositions, laundry detergent compositions and automatic dishwashing compositions comprising one or more amine oxides of formula (I) 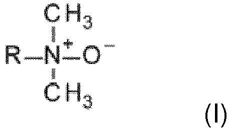 wherein
R is a radical of the formula 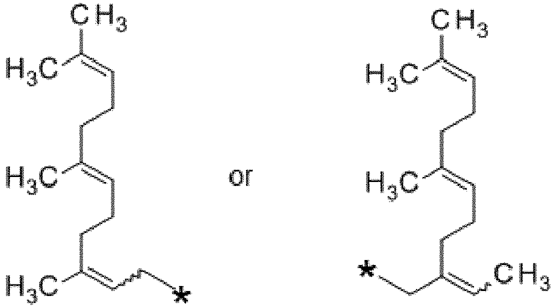
* indicates the binding site and
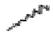 is a bond and indicates that the double bond connected thereto can be either cis or trans in configuration,
preferably an amine oxide of formula (Ia)

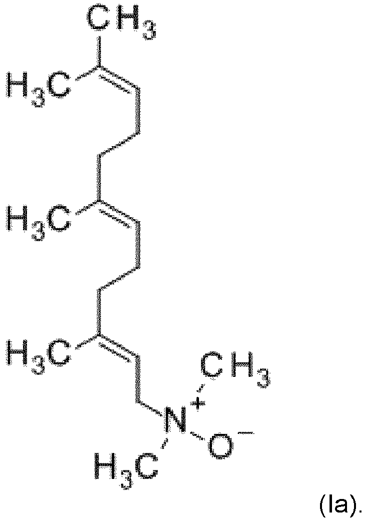

The inventive crop protection compositions, hard surface cleaning compositions, laundry detergent compositions and automatic dishwashing compositions may comprise further ingredients. These further ingredients may be selected from those generally used in such compositions.

The following examples describe the synthesis and evaluation of the inventive amine oxides and/or the inventive compounds. These examples are intended to illustrate the invention without limiting its scope.

### Examples:

The following abbreviations are used:

| abbreviation | meaning |
|---|---|
| acac | acetylacetonate |
| cod | 1,5-cyclooctadiene |
| dba | dibenzylideneacetone |
| DimCarb | dimethylammonium dimethylcarbamate |
| DMF | N,N-dimethylformamide |
| g | gram |
| h | hour |
| hfacac | 1,1,1,5,5,5-hexafluoroacetylacetonate |
| mg | milligram |
| mL | milliliter |
| mmol | millimol |
| mol-% | mol percent |
| rpm | revolutions per minute |
| tfa | trifluoroacetate |
| wt.-% | percent by weight |

### Example 1 - Synthesis of dimethyl farnesyl amine oxide

### Step 1 - Synthesis of dimethyl farnesyl amine

In a 25 mL stainless steel autoclave 3066.4 mg trans-beta-farnesene and 6039.2 mg DimCarb (= dimethylammonium dimethylcarbamate) are added to 10.6 mg Pd(tfa)₂ and 100.0 mg DPPBTS. The autoclave is sealed and heated to 100 °C for 3 h, and stirred with a magnetic stir bar at 500 rpm. To stop the reaction, the autoclave is cooled to room temperature and the developed gas pressure is released. The reaction mixture is transferred into a Schlenk tube under a counter flow of argon. In the Schlenk tube, a spontaneous phase separation occurs. The lower, polar phase is transferred back into the autoclave. The nonpolar product phase is extracted using 1073.8 mg DimCarb. After the phases separate again, the polar phase is transferred into the autoclave together with 3065.7 mg farnesene and the reaction is started again. The product phase may be purified using column chromatography. Between 2543 and 3366 mg of product is obtained in several runs.

### Step 2 - Oxidation of dimethyl farnesyl amine to dimethyl farnesyl amine oxide

In a 100 mL reaction vessel with a magnetic stir bar, dimethyl farnesyl amine (15 g, 0.058 mol) was suspended in deionized water (32.4 g). The vessel was sealed with a cap. Diethylenetriamine-pentaacetic acid pentasodium salt solution (40 wt.-% in water, 0.036 g, 0.028 mmol) was then added. The reaction mixture was heated to 50 °C under CO₂ atmosphere. Hydrogen peroxide (35 wt.-% in water, 5.77 g, 0.059 mol) was added dropwise over a period of 3 h. The reaction was then kept at 50 °C for another 1 h. Amine value was measured to monitor the reaction. Dimethyl farnesyl amine oxide was obtained as a light yellow aqueous solution (30 wt.-%, 38.7 g).

### Comparative example - Synthesis of diethyl farnesyl amine oxide

### Step 1 - Synthesis of diethyl farnesyl amine

10.6 mg Pd(tfa)₂ and 137.8 mg DPEPhos are weighed into a 25 mL stainless steel autoclave and are dissolved in 5 mL methanol. Thereafter, 817.7 mg trans-beta-farnesene and 297.1 mg diethyl amine are added. The autoclave is sealed and pressurized with 5 bar of argon. The autoclave is heated to 100 °C for 5 h and stirred with a magnetic stir bar at 500 rpm. To stop the reaction, the autoclave is cooled to room temperature and thereafter, the argon is cautiously released. The solvent is removed from the reaction solution obtained under reduced pressure and the product is purified using column chromatography. 960.4 mg (89 %) of hydroamination products are obtained.

### Step 2 - Oxidation of diethyl farnesyl amine to diethyl farnesyl amine oxide

This synthesis follows the same procedure as described in Example 1, using 17 g (0.057 mol) diethyl farnesyl amine and 13.63 g (0.15 mol) hydrogen peroxide to produce diethyl farnesyl amine oxide as a suspension, which went quickly into phase separation.

### Example 2 - Evaluation of surfactant properties of dimethyl farnesyl amine oxide and diethyl farnesyl amine oxide

Dimethyl farnesyl amine oxide and diethyl farnesyl amine oxide were tested for their surface tension reduction properties, wetting abilities and foam ability. The products were tested in the methods described below.

### CMC (critical micelle concentration) and surface tension measurements

These measurements were conducted with the Krüss Tensiometer K 100 (Ring), which utilized the Du Noüy ring method. Surface tension profiles of the amine oxides were measured at 25 °C.

### Determination of wetting ability by immersion

This measurement was modified based on the European Standard EN 1772:2000. A 500 mL 0.1 wt.-% aqueous solution of test surfactant was prepared. The test solution was kept at room temperature to stabilize for 1 h. A raw cotton disc (30 mm diameter, wfk Testgewebe GmbH), was clamped in a gripper and immersed in the solution. Stopwatch was started at the moment when the lower part of the disc touched the solution and stopped when the disc began to sink of its own accord. The arithmetic mean of five measurements was calculated and recorded as the wetting time.

### Measurement of foam creation and decay on a SITA foam tester

This method monitors foam generation and decay at room temperature over time. A surfactant solution of 0.01 g/L was pumped into the SITA foam tester R2000. The speed of the stirring plate was set to 1200 rpm. Foam creation was recorded in a 10 second interval for 5 minutes and decay was recorded in a 30 second interval for 15 minutes at room temperature.

Table 1 shows a comparison of the properties of dimethyl farnesyl amine oxide and diethyl farnesyl amine oxide.

**Table 1 Properties of dimethyl farnesyl amine oxide and diethyl farnesyl amine oxide**

| Dialkyl farnesyl amine oxide | CMC (g/L) | C₂₀ (g/L) | Surface tension (mN/m) | Wetting time (seconds) | Foam ability |
|---|---|---|---|---|---|
| Dimethyl | 1.09 | 0.018 | 28.7 | 28 | No foam was generated after 5 minutes |
| Diethyl (comparative) | No correct measurement was able to be obtained due to fast phase separation | | | 54 | |

| | | | | | |
|---|---|---|---|---|---|
| C₂₀: concentration of surfactant (here: dialkyl farnesyl amine oxide) to reduce the surface tension of water by 20 mN/m | | | | | |

Diethyl farnesyl amine oxide has very poor solubility in water. This made the measurement of surface tension impossible and resulted in a longer wetting time. However, dimethyl farnesyl amine oxide on the other hand showed very effective surface tension reduction properties and a good wetting time, while producing no foam at all.

### Example 3 - Comparison of dynamic surface tension

### Dynamic surface tension measurements

These measurements were conducted with the Krüss PocketDyne BP2100, which utilized the maximum bubble pressure method of surface tension analysis. Surface tension profiles of dimethyl farnesyl amine oxide and diethyl farnesyl amine oxide at the desired concentration were measured in deionized water at 25 °C.

When surfactants are used in crop protection applications, usually 0.1 - 10 g/L, preferably 0.3 - 3 g/L, of each surfactant is present, and the average time for surfactant solution spraying from nozzle to crop leaves is between 20 to 400 ms (ms: milliseconds).

Table 2 shows the results of a comparison of the dynamic surface tension at surface ages of 20 ms, 50 ms, 100 ms, 200 ms and 400 ms for dimethyl farnesyl amine oxide and diethyl farnesyl amine oxide. The results in table 2 are given in mN/m.

**Table 2 Surface tension of dimethyl farnesyl amine oxide and diethyl farnesyl amine oxide at different surface ages**

| Dialkyl farnesyl amine oxide | Concentration (g/L) | 20 ms | 50 ms | 100 ms | 200 ms | 400 ms |
|---|---|---|---|---|---|---|
| Dimethyl | 0.3 | 59.9 | 56.9 | 52.9 | 51.5 | 49.5 |
| | 1.0 | 43.8 | 40.7 | 39.3 | 38.1 | 36.9 |
| | 3.0 | 35.3 | 33.2 | 32.5 | 32.1 | 31.5 |
| Diethyl (comparative) | 0.3 | 66.7 | 65.6 | 64.3 | 65.7 | 65.0 |
| | 1.0 | 60.0 | 62.0 | 57.3 | 56.0 | 54.9 |
| | 3.0 | 55.2 | 52.7 | 51.8 | 50.6 | 51.6 |

In general, if a surfactant compound can reduce the surface tension below 55 mN/m at the surface age of 200 ms, it is considered as a good wetting agent in crop protection applications. The data of table 2 show that dimethyl farnesyl amine oxide is a significantly better wetting agent than diethyl farnesyl amine oxide.

The evaluation of dimethyl farnesyl amine oxide and diethyl farnesyl amine oxide revealed significant differences in application tests, in which dimethyl farnesyl amine oxide showed better surface tension reduction, better wetting ability and no foam.

## Claims

1. Amine oxide of the formula (I) 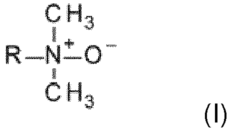 wherein
R is a radical of the formula 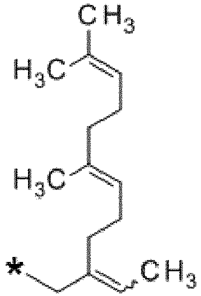 wherein
* indicates the binding site and
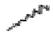 is a bond and indicates that the double bond connected thereto can be either cis or trans in configuration
or
R is a radical of the formula 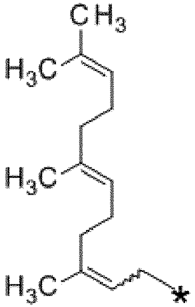 wherein
* indicates the binding site and
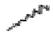 is a bond and indicates that the double bond connected thereto is cis in configuration.

2. Process for the preparation of an amine oxide according to claim 1, comprising reacting a dimethyl farnesyl amine with hydrogen peroxide in the presence of carbon dioxide.

3. Process according to claim 2, **characterized in that** the reaction is performed in a solvent, preferably in water as the solvent or in a solvent comprising water and one or more further solvents different from water, and more preferably in water as the solvent, at a temperature of from 20 to 100 °C, preferably of from 30 to 70 °C, and furthermore preferably at atmospheric pressure.

4. Process according to claim 2 or 3, **characterized in that** the molar ratio dimethyl farnesyl amine : hydrogen peroxide is of from 1 : 1 to 1 : 50, preferably of from 1 : 1 to 1 : 10 and even more preferably of from 1 : 1 to 1 : 3.

5. Process according to one or more of claims 2 to 4, **characterized in that** the dimethyl farnesyl amine is obtained by reacting farnesene with dimethyl amine or a dimethyl amine source in the presence of a transition metal catalyst.

6. Process according to claim 5, **characterized in that** the dimethyl amine source is selected from the group consisting of dimethyl amine hydrochloride, preferably to be used together with a base, and dimethyl ammonium dimethyl carbamate.

7. Process according to claim 5 or 6, **characterized in that** the transition metal catalyst comprises a metal selected from group 10 of the periodic table and preferably selected from the group consisting of nickel, palladium and platinum.

8. Process according to one or more of claims 5 to 7, **characterized in that** the molar ratio farnesene : (dimethyl amine or dimethyl amine source) is of from 1 : 1 to 1 : 10.

9. Process according to one or more of claims 5 to 8, **characterized in that** the reaction is performed at a temperature of from 50 to 150 °C, preferably of from 60 to 120 °C and even more preferably of from 70 to 100 °C.

10. Process according to one or more of claims 5 to 9, **characterized in that** the farnesene is beta-farnesene.

11. Use of an amine oxide of formula (I) 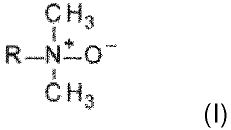 wherein
R is a radical of the formula 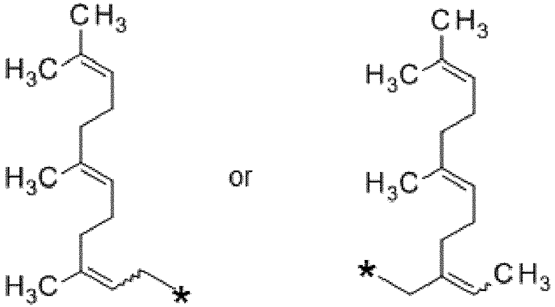
* indicates the binding site and
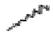 is a bond and indicates that the double bond connected thereto can be either cis or trans in configuration,
as a surfactant.

12. Use of an amine oxide of formula (I) 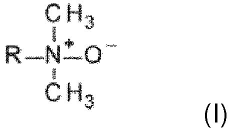 wherein
R is a radical of the formula 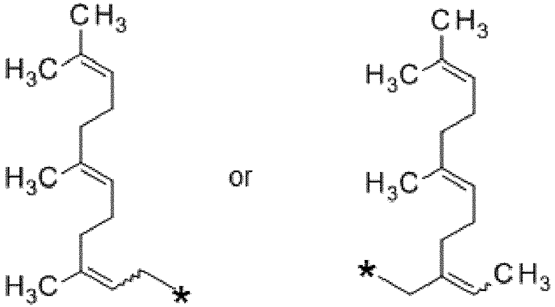
* indicates the binding site and
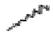 is a bond and indicates that the double bond connected thereto can be either cis or trans in configuration,
as a wetting agent.

13. Use according to claim 11 or 12, wherein the amine oxide of formula (I) has the formula (Ia)

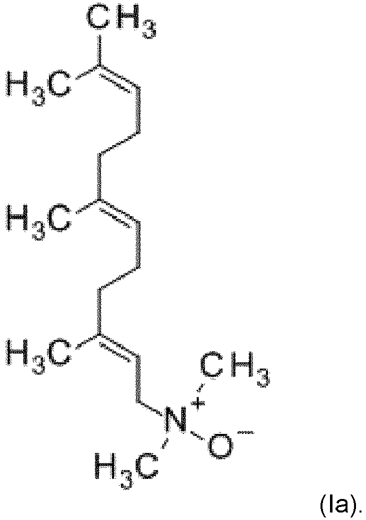

14. Crop protection composition, hard surface cleaning composition, laundry detergent composition or automatic dishwashing composition comprising one or more amine oxides of formula (I) 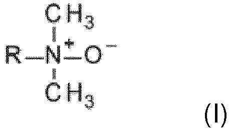 wherein
R is a radical of the formula 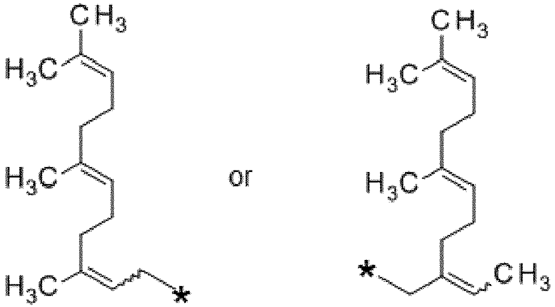
* indicates the binding site and
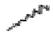 is a bond and indicates that the double bond connected thereto can be either cis or trans in configuration.

15. Crop protection composition, hard surface cleaning composition, laundry detergent composition or automatic dishwashing composition according to claim 14, wherein the amine oxide of formula (I) has the formula (Ia)

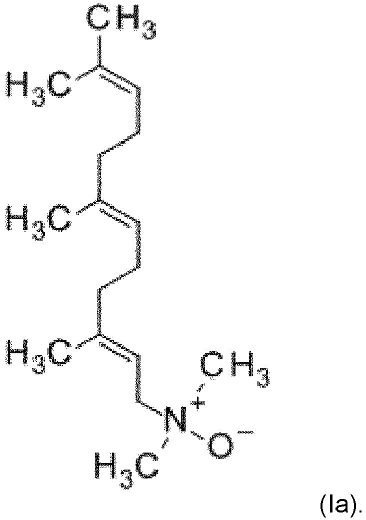

## Patentansprüche

1. Aminoxid der Formel (I) 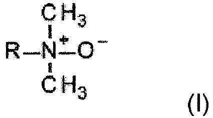 wobei
R für einen Rest der Formel 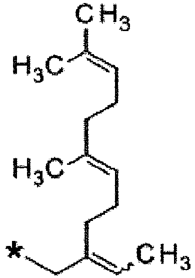 steht, wobei
* für die Bindungsstelle steht und
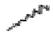 für eine Bindung steht und anzeigt, dass die daran gebundene Doppelbindung entweder cis- oder trans-Konfiguration aufweisen kann,
oder
R für einen Rest der Formel 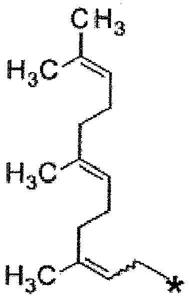 steht, wobei
* für die Bindungsstelle steht und
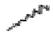 für eine Bindung steht und anzeigt, dass die daran gebundene Doppelbindung cis-Konfiguration aufweist.

2. Verfahren zur Herstellung eines Aminoxids nach Anspruch 1, bei dem man ein Dimethylfarnesylamin in Gegenwart von Kohlendioxid mit Wasserstoffperoxid umsetzt.

3. Verfahren nach Anspruch 2, **dadurch gekennzeichnet, dass** die Umsetzung in einem Lösungsmittel, vorzugsweise in Wasser als Lösungsmittel oder in einem Lösungsmittel, das Wasser und ein oder mehrere weitere Lösungsmittel, die von Wasser verschieden sind, umfasst, und weiter bevorzugt in Wasser als Lösungsmittel bei einer Temperatur von 20 bis 100 °C, vorzugsweise von 30 bis 70 °C, und weiterhin vorzugsweise bei Normaldruck durchgeführt wird.

4. Verfahren nach Anspruch 2 oder 3, **dadurch gekennzeichnet, dass** das Molverhältnis von Dimethylfarnesylamin zu Wasserstoffperoxid 1:1 bis 1:50, vorzugsweise 1:1 bis 1:10 und noch weiter bevorzugt 1:1 bis 1:3 beträgt.

5. Verfahren nach einem oder mehreren der Ansprüche 2 bis 4, **dadurch gekennzeichnet, dass** das Dimethylfarnesylamin durch Umsetzung von Farnesen mit Dimethylamin oder einer Dimethylaminquelle in Gegenwart eines Übergangsmetallkatalysators erhalten wird.

6. Verfahren nach Anspruch 5, **dadurch gekennzeichnet, dass** die Dimethylaminquelle aus der Gruppe bestehend aus Dimethylaminhydrochlorid, das vorzugsweise zusammen mit einer Base zu verwenden ist, und Dimethylammoniumdimethylcarbamat ausgewählt wird.

7. Verfahren nach Anspruch 5 oder 6, **dadurch gekennzeichnet, dass** der Übergangsmetallkatalysator ein Metall umfasst, das aus Gruppe 10 des Periodensystems ausgewählt ist und vorzugsweise aus der Gruppe bestehend aus Nickel, Palladium und Platin ausgewählt ist.

8. Verfahren nach einem oder mehreren der Ansprüche 5 bis 7, **dadurch gekennzeichnet, dass** das Molverhältnis von Farnesen zu Dimethylamin oder Dimethylaminquelle 1:1 bis 1:10 beträgt.

9. Verfahren nach einem oder mehreren der Ansprüche 5 bis 8, **dadurch gekennzeichnet, dass** die Umsetzung bei einer Temperatur von 50 bis 150 °C, vorzugsweise von 60 bis 120 °C und noch weiter bevorzugt von 70 bis 100 °C durchgeführt wird.

10. Verfahren nach einem oder mehreren der Ansprüche 5 bis 9, **dadurch gekennzeichnet, dass** es sich bei dem Farnesen um beta-Farnesen handelt.

11. Verwendung eines Aminoxids der Formel (I) 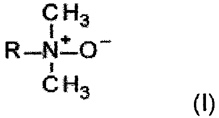 wobei
R für einen Rest der Formel 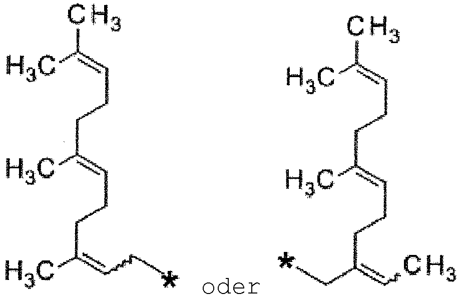 steht,
* für die Bindungsstelle steht und
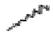 für eine Bindung steht und anzeigt, dass die daran gebundene Doppelbindung entweder cis- oder trans-Konfiguration aufweisen kann,
als Tensid.

12. Verwendung eines Aminoxids der Formel (I) 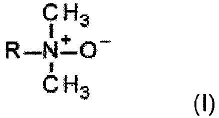 wobei
R für einen Rest der Formel 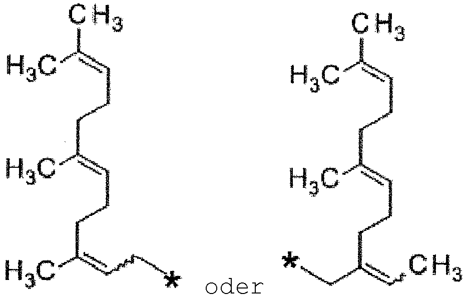 steht,
* für die Bindungsstelle steht und
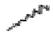 für eine Bindung steht und anzeigt, dass die daran gebundene Doppelbindung entweder cis- oder trans-Konfiguration aufweisen kann,
als Netzmittel.

13. Verwendung nach Anspruch 11 oder 12, wobei das Aminoxid der Formel (I) die Formel (Ia) aufweist:

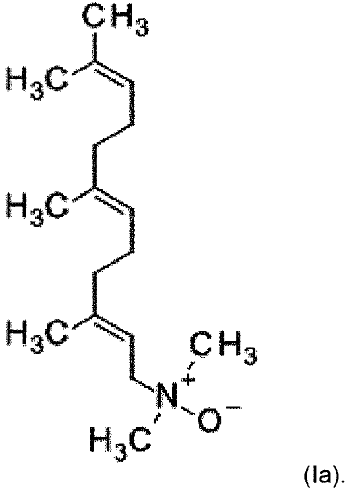

14. Pflanzenschutzzusammensetzung, Reinigungsmittel für harte Oberflächen, Waschmittelzusammensetzung oder Geschirrspülmaschinenzusammensetzung, umfassend ein oder mehrere Aminoxide der Formel (I) 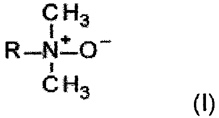 wobei
R für einen Rest der Formel 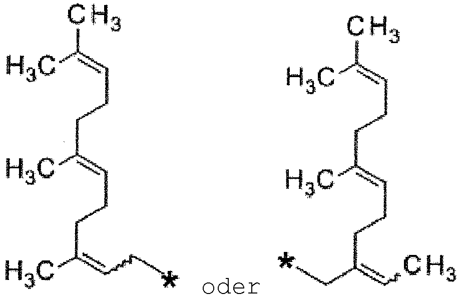 steht,
* für die Bindungsstelle steht und
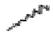 für eine Bindung steht und anzeigt, dass die daran gebundene Doppelbindung entweder cis- oder trans-Konfiguration aufweisen kann.

15. Pflanzenschutzzusammensetzung, Reinigungsmittel für harte Oberflächen, Waschmittelzusammensetzung oder Geschirrspülmaschinenzusammensetzung nach Anspruch 14, wobei das Aminoxid der Formel (I) die Formel (Ia) aufweist:

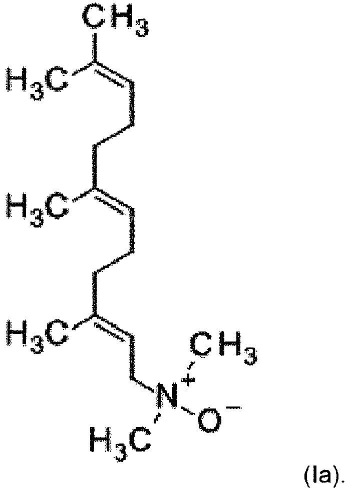

## Revendications

1. Oxyde d'amine de formule (I) 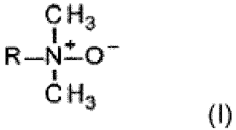 R étant un radical de formule 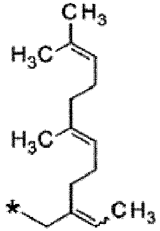
* indiquant le site de liaison et
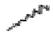 étant une liaison et indiquant que la double liaison reliée à celle-ci peut être soit cis, soit trans en configuration
ou
R étant un radical de formule 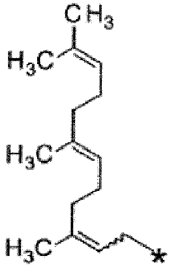
* indiquant le site de liaison et
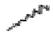 étant une liaison et indiquant que la double liaison reliée à celle-ci est cis en configuration.

2. Procédé pour la préparation d'un oxyde d'amine selon la revendication 1, comprenant la mise en réaction d'une diméthylfarnésylamine avec du peroxyde d'hydrogène en présence de dioxyde de carbone.

3. Procédé selon la revendication 2, **caractérisé en ce que** la réaction est réalisée dans un solvant, préférablement dans de l'eau en tant que solvant ou dans un solvant comprenant de l'eau et un ou plusieurs solvants supplémentaires différents de l'eau, et plus préférablement dans l'eau en tant que solvant, à une température allant de 20 à 100 °C, préférablement allant de 30 à 70 °C, et de plus préférablement à pression atmosphérique.

4. Procédé selon la revendication 2 ou 3, **caractérisé en ce que** le rapport molaire diméthylfarnésylamine : peroxyde d'hydrogène est de 1:1 à 1:50, préférablement de 1:1 à 1:10 et encore plus préférablement de 1:1 à 1:3.

5. Procédé selon l'une ou plusieurs des revendications 2 à 4, **caractérisé en ce que** la diméthylfarnésylamine est obtenue par la mise en réaction de farnésène avec de la diméthylamine ou une source de diméthylamine en présence d'un catalyseur de métal de transition.

6. Procédé selon la revendication 5, **caractérisé en ce que** la source de diméthylamine est choisie dans le groupe constitué par le chlorhydrate de diméthylamine, préférablement devant être utilisé avec une base, et le diméthylcarbamate de diméthylammonium.

7. Procédé selon la revendication 5 ou 6, **caractérisé en ce que** le catalyseur de métal de transition comprend un métal choisi parmi le groupe 10 du tableau périodique et préférablement choisi dans le groupe constitué par le nickel, le palladium et le platine.

8. Procédé selon l'une ou plusieurs des revendications 5 à 7, **caractérisé en ce que** le rapport molaire farnésène : (diméthylamine ou source de diméthylamine) est de 1:1 à 1:10.

9. Procédé selon l'une ou plusieurs des revendications 5 à 8, **caractérisé en ce que** la réaction est réalisée à une température allant de 50 à 150 °C, préférablement allant de 60 à 120 °C et encore plus préférablement allant de 70 à 100 °C.

10. Procédé selon l'une ou plusieurs des revendications 5 à 9, **caractérisé en ce que** le farnésène est le bêta-farnésène.

11. Utilisation d'un oxyde d'amine de formule (I) 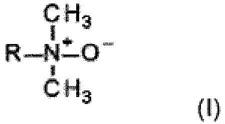 R étant un radical de formule 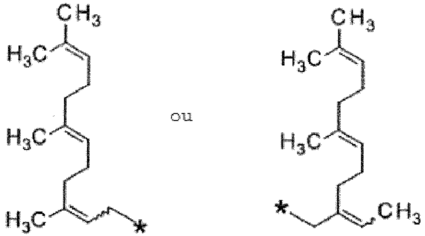
* indiquant le site de liaison et
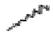 étant une liaison et indiquant que la double liaison reliée à celle-ci peut être soit cis, soit trans en configuration,
en tant que tensioactif.

12. Utilisation d'un oxyde d'amine de formule (I) 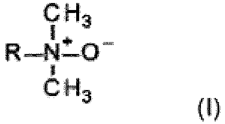 R étant un radical de formule 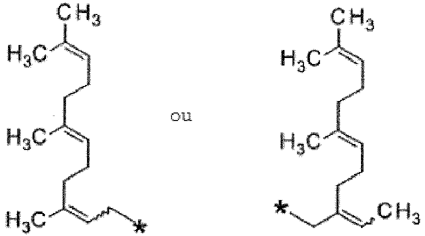
* indiquant le site de liaison et
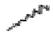 étant une liaison et indiquant que la double liaison reliée à celle-ci peut être soit cis, soit trans en configuration,
en tant qu'agent mouillant.

13. Utilisation selon la revendication 11 ou 12, l'oxyde d'amine de formule (I) possédant la formule (Ia)

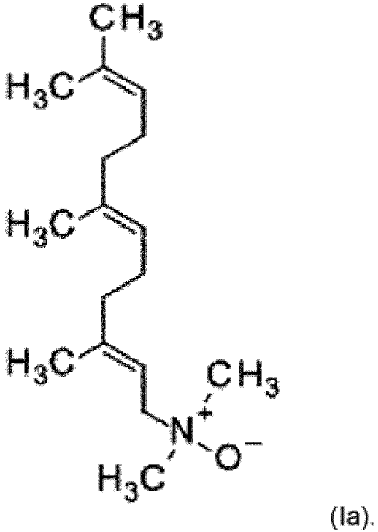

14. Composition de protection de culture, composition de nettoyage de surface dure, composition de détergent de lavage ou composition pour lave-vaisselle automatique comprenant un ou plusieurs oxydes d'amine de formule (I) 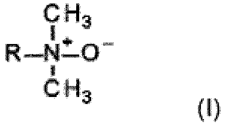 R étant un radical de formule 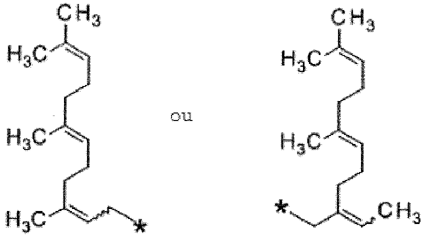
* indiquant le site de liaison et
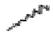 étant une liaison et indiquant que la double liaison reliée à celle-ci peut être soit cis, soit trans en configuration.

15. Composition de protection de culture, composition de nettoyage de surface dure, composition de détergent de lavage ou composition pour lave-vaisselle automatique selon la revendication 14, l'oxyde d'amine de formule (I) possédant la formule (Ia)
